# EUROPÄISCHE PATENTSCHRIFT

(11) **EP 0 674 889 B1**
(45) Veröffentlichungstag und Bekanntmachung des Hinweises auf die Patenterteilung: **09.12.1998**
(21) Anmeldenummer: 95103917.1
(22) Anmeldetag: 17.03.1995
(51) Int. Cl.: A61F 5/01, A61F 5/058

(54) **Knieschiene**
Knee-brace
Attelle de genou

(30) Priorität: 01.04.1994 DE 4411469
(43) Veröffentlichungstag der Anmeldung: 04.10.1995
(73) Patentinhaber: Beiersdorf Aktiengesellschaft, 20245 Hamburg (DE)
(72) Erfinder: Brandt, Dieter, D-40545 Düsseldorf (DE); Szlema, Ingeborg M. A., D-47906 Kempen (DE); Bodenschatz, Stefan, D-21614 Buxtehude (DE)
(74) Vertreter: Richter, Werdermann & Gerbaulet

(56) Entgegenhaltungen:
- EP-A- 0 496 071
- DE-A- 3 925 007
- US-A- 3 831 467
- US-A- 4 111 194
- US-A- 4 505 269

## Beschreibung

Die Erfindung betrifft eine Knieschiene mit einer am Bein anzulegenden, auf der Vorderseite des Beines zusammengehaltenen Hülse.

Operative Eingriffe am Knie verlangen eine postoperative Ruhigstellung, die klassisch durch einen Funktionsgipsverband hergestellt wird. Typischerweise sind hier Kunststoff-Gipse oder Schienen anzutreffen, die mit elastischen Binden angewinkelt werden. Bei mobilen Patienten werden diese Binden häufig durch Klettbänder ersetzt, die direkt auf schalenförmigen Schienen angebracht werden. Zur Erhöhung des Tragekomforts werden diese Schalen oftmals ausgepolstert. Durch die DE-A-31 23 148 ist eine Knieschiene mit einem flexiblen Mantel bekannt, dessen Seitenränder nach dem Anlegen des Mantels an das Bein auf der Vorderseite des Beines zusammengehalten werden und je eine Aussparung für die Kniescheibe aufweisen, wobei längs dieser Seitenränder verstellbare Verschlußelemente vorgesehen sind und wobei der Mantel an den Bereichen, die nach dem Anlegen des Mantels an das Bein an den Seitenflächen und der Rückseite des Beines zu liegen kommen, mit Versteifungsstäben versehen ist. Der Mantel dieser Knieschiene ist aus mehreren anatomiegerechten Zuschnitten zusammengesetzt, wobei die Zuschnitte und die Versteifungsstäbe eine zur Fixierung des Kniegelenks in einer Beugestellung von etwa 20° bestimmte Form und Krümmung aufweisen.

Auch die Knieschiene nach der DE-A-42 29 044 besteht aus einem flexiblen Mantel, der an seinen Seitenrändern mit Verschlußelementen versehen ist, die den Mantel nach dem Anlegen an das Bein in Form einer geschlossenen Röhre zusammenhalten, wobei die Längsachse des den Unterschenkel umgebenden Teils eine Winkelstellung zur Längsachse des den Oberschenkel umgebenden Teils einnimmt. Bei dieser Knieschiene ist der den Mantel bildende Zuschnitt einteilig ausgebildet und in eine Ebene abwickelbar, wobei in abgewickeltem Zustand eine sich im wesentlichen parallel zur Beinlängsachse erstreckende Ausnehmung vorgesehen ist, welche längs der Beinrichtung größere Abmessungen aufweist als quer dazu. Die Ausnehmung erreicht nicht an mindestens einem ihrer Enden die jeweils nächstliegende der Kanten des Zuschnittes. Die beiden längsgerichteten Seitenkanten der Ausnehmung sind bei fertiggestellter Knieschiene miteinander verbunden.

In beiden Fällen bestehen die Knieschienen aus einem flexiblen Mantel. Ist dieser Mantel aus mehreren Zuschnitten zusammengesetzt, so ist stets eine große Genauigkeit bei der Näharbeit erforderlich. Ein ungenaues Aneinanderliegen und Zusammenfügen der einzelnen Zuschnitte kann zu einer fehlerhaften Form und damit zu einem schlechten Sitz der Schiene führen. Beide bekannten Knieschienen sind so ausgebildet, daß sie das Kniegelenk in einer vorgegebenen Beugestellung von etwa 20° fixieren, wobei eine im Verlauf der Behandlung auftretende Lockerung der Knieschiene korrigierbar ist. Um diese vorgegebene Beugestellung einhalten zu können, sind die Mäntel der Knieschienen aus mehreren anatomiegerechten Zuschnitten zusammengesetzt, wobei die Zuschnitte und die Versteifungsstäbe eine zur Fixierung des Kniegelenks in einer Beugestellung von etwa 20° bestimmte Form und Krümmung aufweisen. Aufgrund der vorgegebenen, die Knieschienen bildenden Mäntel ergeben sich oftmals Paßformprobleme und Anpassungsschwierigkeiten. Letztlich müssen diese bekannten Knieschienen maßgefertigt sein. Hinzu kommt, daß auch bei stillgelegtem Knie der Patient mit den bekannten Knieschienen keine Streckübungen machen kann. Da die Mäntel dieser Knieschienen im angelegten Zustand das Bein allseitig umhüllen, ist ein Wärmestau nicht vermeidbar.

Durch die US-A- 3.831.467 ist eine Knieorthese bekannt, die aus einem elastischen Flächengebilde in Form eines Gewebezuschnittes besteht, das hüllenartig im Kniebereich am Ober- und Unterschenkel gelegt und mittels einer Vielzahl von Verschlußbändern am Bein festgehalten wird. Das Bein wird im angelegten Zustand der Knieorthese von dieser ganz umschlossen; nur eine Öffnung für die Patella wird freigelassen. Zur Versteifung der Orthese, durch die ein Beugen des Knies verhindert werden soll, sind in das Flächengebilde Versteifungsstäbe integriert, von denen die mittleren drei Versteifungsstäbe gegenüber der Patella, also die direkt hinter der Kniekehle angeordneten Versteifungsstäbe, die wichtigtsten sind. Zusätzlich kann seitlich noch je ein Versteifungsstab vorgesehen sein. Wegen des natürlichen Hohlraumes der Kniekehle liegen die Versteifungsstäbe dort nicht am Bein an, was ein Hin- und Herrutschen der Orthese am Bein bewirken kann, auch wenn die Verschlußbänder fest angezogen sind. Aus diesem Grunde ist ein weiches Polster auf der inneren Oberfläche der Knieorhtese an dieser Stelle angebracht. Dieses Polster füllt lediglich die Kniekehle aus; es hält jedoch das Knie nicht in der für eine erfolgreiche Behandlung erforderliche Beugehaltung von 10° bis 20°, da das Polster relativ flach ausgebildet ist und lediglich zur Verminderung des Verrutschens der angelegten Orthese beitragen soll.

Dadurch, daß das Flächengebilde im angelegten Zustand das Bein hüllenartig umschließt, läßt sich durch die geschlossene Bauweise dieser Knieorthese ein Wärmestau nicht verhindern, der bei höheren Außentemperaturen oder bei Bewegungen auftritt und damit zu einem Transpirieren des betroffenen Beines führen kann. Neben dem für den Patienten unangenehmen Gefühl wird durch den die Haut beeinträchtigenden Schweiß die Schutzfunktion der Haut verringert; Hautrötungen und Juckreiz sind die Folgen. Eventuelle Infektionen finden dann ein gutes Klima vor, das eine Verbreitung dieser fördern oder zumindest eine Abheilung erschwerden kann. Wegen der geschlossenen Bauweise dieser Knieorthese ist eine Kontrolle des Beines ohne Abnahme der Knieorthese nicht möglich.

Durch die US-A- 4,111,194 ist eine Knieorthese bekannt, die aus einer am Bein anlegbaren Halbschale mit im Bereich deren Längsränder angeformten teilringförmigen und das Bein im angelegten Zustand Vorderseitig umgreifenden Haltezungen, die mit Verschlußmitteln versehen sind, um der Knieorthese am Bein den erforderlichen Halt zu geben. Die Halbschale besteht aus einem eine hohe Eigensteifigkeit aufweisenden Kunststoff, um dem Bein einen festen Halt zu geben. Innenwandseitig ist die Halbschale mit einem Kissen versehen, um das Knie in einer vorgegebenen Beugehaltung zu halten. Auch bei dieser Knieorthese sind im angelegten Zustand Wärmestaus nicht vermeidbar, da die Halbschale im angelegtem Zustand etwa den halben Umfang des Beines umgreift.

Aufgabe der vorliegenden Erfindung ist es, eine postoperative Knieschiene zur Ruhigstellung des Kniegelenkes zweckmäßigerweise in funktionaler leichter Beugestellung zu schaffen, die es dem Patienten ermöglicht, bei angelegter Knieschiene Streckübungen in der Schiene vorzunehmen, die eine individuelle Anpassung an verschiedene Beingrößen und -abmessungen sowie Beinkonturen ermöglicht, eine wärmestauvermeidende Konstruktion und einen hohen Tragekomfort aufweist.

Gelöst wird diese Aufgabe durch die im Anspruch 1 angegebenen Merkmale.

Die die erfindungsgemäße Knieschiene bildende Hülse kann auf ihrer Innenseite im Kniekehlenbereich ein bei angelegter Knieschiene das Knie in einer vorgegebenen Beugehaltung von etwa 10° bis 20°,zweckmäßigerweise von 15° bis 20°,haltendes Kissen aus einem formstabilen, kompressiblen und ein Rückstellvermögen aufweisenden Kunststoff, zweckmäßigerweise einem Schaumkunststoff oder einem anderen geeigneten Material aufweisen, wobei das Kissen auch abnehmbar ist, um die Knieschiene auch dann einsetzen zu können, wenn das Bein in gestreckter Lage gehalten werden soll.

Dieses Kniebeugehaltekissen besteht aus einem etwa halbkreisförmigen Formkörper mit einer das Bein in seinem Kniekehlenbereich abstützenden Breite. Durch die Form und die Abmessungen des Kissens wird die gewünschte und erforderliche Beugehaltung erreicht.

Bevorzugterweise ist das Kniebeugehaltekissen so bemessen, daß in dessen Längsquerschnittsfläche die Fläche eines mit seiner Basis auf der Kissenstandfläche aufliegenden, gleichschenkligen, rechtwinkligen Dreiecks mit einer dem Radius des halbkreisförmigen Formkörpers entsprechenden Mittellinie legbar ist.

Die Hülse der Knieschiene besteht zur Ausbildung einer flexiblen, jedoch stabilen Halbschale aus zwei stabilen, eine hohe Eigensteifigkeit aufweisenden Seitenteilen, die an ihren beiden Enden über je ein in der Länge verstellbares Verschlußband miteinander und mittig über einen Verbindungssteg mit in dessen seitlichen Bereich an den beiden Seitenteilen befestigten, sich zu einem mittleren Verschlußband miteinander verbindbaren Verschlußbandteilen verbunden sind, wobei an dem etwa mittigen Verbindungssteg das Kniebeugehaltekissen angeordnet ist, das in Knieschienenlängsrichtung verläuft und das mindestens eine der Kniebreite entsprechende Breite aufweist. Das Kniebeugehaltekissen ist lösbar auf der Innenseite des mittleren Verbindungssteges der schalenförmigen Hülse angeordnet und auf dem mittleren Verbindungssteg lageveränderbar gehalten, so daß eine individuelle Anpassung an den Kniebeugebereich des Beines und dessen Anatomie möglich ist.

Die Seitenteile der Hülse weisen als stabilisierende Elemente mindestens je einen in Knieschienenlängsrichtung verlaufenden, rohrförmigen Stab auf, wobei bevorzugterweise in jedem Seitenteil der Hülse drei rohrförmige Stäbe vorgesehen sind, die parallel zueinander liegend sind. Die Anordnung der einzelnen rohrförmigen Stäbe erfolgt in taschenförmigen Kammern, die in jedem Seitenteil der Hülse ausgebildet sind, wozu jedes Seitenteil aus einem doppelwandig ausgebildeten Gewebestreifen besteht.

Bevorzugterweise sind die rohrförmigen Stäbe eines jeden Seitenteils der Hülse gerade verlaufend mit Abwinkelungen im unteren Wadenbereich ausgebildet. Die rohrförmigen Stäbe bestehen aus einem Leichtmetall, zweckmäßigerweise aus Aluminium, glasfaserverstärktem Kunststoff oder sind als Kohlefaserstäbe ausgebildet.

Eine V-förmig und konisch sich in Richtung zum Wadenbereich verlaufende Ausgestaltung der beiden Seitenteile der Hülse ermöglicht eine Anpassung an die anatomischen und biomechanischen Verhältnisse.

Es ist hiernach eine formgenähte Knie-Hülse als funktionelle Knielagerungsschiene zur Stabilisierung und Ruhigstellung des Kniegelenks in funktionaler leichter Beugestellung geschaffen. Die Knieschiene besteht aus seitlich angeordneten stabilisierenden Stäben, die über Verschlußbänder miteinander verbunden sind, wobei diese Verschlußbänder auf der Bein-Vorderseite durch überlappende Klettverschlüsse oder anderweitig ausgebildete Verschlüsse, wie Schnallen od.dgl.,verschließbar sind.

Das Kniebeugehaltekissen ist in der halbschalenförmig ausgebildeten Hülse aus formstabilem Schaumstoff gefertigt, um das Knie in die gewünschte leichte Beugehaltung von etwa 10° bis 20° zu bringen. Der eingesetzte Schaumstoff ist bei Kraft- und Druckanwendung kompressibel, so daß der Patient bei angelegter Knieschiene Streckübungen in der Lagerungsschiene vornehmen kann. Ohne Kraftausübung wird das Bein wieder in eine leichte Beugehaltung zurückversetzt. Das Kissen mit polsterartiger Wirkung wird mit einem Klettband variabel, d.h. längenverstellbar, an der Innenseite der schalenförmigen Hülse befestigt, um individuell richtig plaziert werden zu können. Beim aufrechten Patienten verhindert das Kissen durch Abstützung an der Unterschenkelmuskulatur zudem das Rutschen der Knieschiene.

Nach dem bei schweren Knie-Orthesen auf dem Markt allgemein gebräuchlichem Prinzip läßt sich eine Stabilisierung des Knies durch Fixierung an drei Punkten erreichen:

Zwei Stellen an der Rückseite des Beines, am Ober- und Unterschenkel, eine Stelle an der Vorderseite des Beines, an der Tibia knapp unterhalb der Patella.Dementsprechend sind die rückwärtigen Verschlußbänder am Ober- und Unterschenkel sowie das vordere,mittige Verschlußband kräftig ausgebildet und fest mit den seitlichen Stabilisierungselementen verbunden. Diese feste Verankerung ist entscheidend für die Funktionsfähigkeit der Knieschiene.

Die seitlichen Stabilisierungselemente bzw. die seitlichen rohrförmigen Stäbe geben den Beugewinkel des Knies nicht vor, sondern sind bevorzugterweise gerade ausgebildet mit einer Abwinklung im unteren Wadenbereich zur besseren Anpassung an die Anatomie. Diese Stabilisierungsstäbe sind nachformbar zur individuellen Anpassung der Knieschiene.

Bei den bekannten Knieschienen ist das Gegenteil der Fall. Soweit Schienen verwendet werden, geben hier die Schienen den Beugewinkel vor und der Formkörper der Knieschiene folgt diesem. Das erfordert aufwendiges Formschneidern und verursacht Paßformprobleme. Bei der erfindungsgemäßen Knieschiene wird dies bei gerader Knieschienenform mit dem eingelegten Kniebeugehaltekissen einfach gelöst.

Die seitlichen Stabiliserungsstäbe der Hülse sind rund ausgebildet, mehrfach angeordnet und besitzen so bei leichtem Gewicht und einfacher Konstruktion eine sehr gute stabilisierende Wirkung.

Die Knieschiene besteht somit aus einer Grundkonstruktion mit zwei stabilen Seitenteilen, die unten und oben durch Verschlußbänder miteinander verbunden sind. Durch diese Konstruktion entsteht eine flexible, jedoch stabile Halbschale, in die das Bein gelegt werden kann. Nach Anbringen der Verschlüsse wird eine das Bein umgebende Hülse geschaffen. Zur Beugung des Knies wird in dieser halbschalenartig ausgebildeten Hülse das Kniekissen angebracht. Die Stabilisierung des Knies wird durch das vordere Verschlußband unterhalb der Patella in Verbindung mit dieser halbschalenartigen Hülse erreicht.

Mit der erfindungsgemäß ausgebildeten Knieschiene werden folgende Vorteile erreicht:
- Eine sehr offene Konstruktion ohne Wärmestau.
- Eine gute Stabilisierung des Knies.
- Einfache Handhabung durch wenige Verschlüsse.
- Gute Paßform und geringes Gewicht.
- Durch das Kissen in der Kniekehle , das sich an der Wade abstützt, wird das Abrutschen der Knieschiene am Bein nach unten verhindert.
- Das Strecken des Beins aus der durch das Kissen vorgegebenen Beugung ist durch die Nachgiebigkeit des Kniebeugehalte- bzw. Kniekehlenkissens möglich, z.B. zur Krankengymnastik.
- Die Knieschiene kann problemlos am liegenden Patienten geöffnet werden; das Bein wird durch das Kissen ähnlich einer Knierolle gebeugt gelagert.
- Durch die Rohre in den Seitenteilen anstelle eines stabilen Stabs formt sich die Knieschiene dem Bein besser an.
- Die Stabilisierungsrohre können einfach gebogen werden, um die Knieschiene, falls erforderlich, genau anzupassen.
- Die Operationswunden werden nicht von einer Bandage abgedeckt.

Als postoperative Knieschiene bietet diese eine hohe Therapiesicherheit. Das Knie ist immobilisierbar und definiert zu stabilisieren. Ein anhaltender, gleichbleibender Stabilisierungseffekt wird erreicht. Das Knie wird in einer definierten, leichten Beugestellung ruhiggestellt, die das vordere Kreuzband entspannt. Die Knieschiene sitzt gut am Bein an und ist drehstabil verankert. Eine gute Weichteilabstützung wird am Unterschenkel erreicht. Die Knieschiene läßt sich unterschiedlichen Ober- und Unterschenkelweiten anpassen.

Mit dieser Knieschiene wird ein hoher Tragekomfort erhalten, denn die Knieschiene scheuert nicht, schnürt nicht ein, trägt wenig auf, ist sehr leicht aufgrund des geringen Gewichtes; sie ist hautfreundlich, luft- und wasserdampfdurchlässig und hat keine unangenehme Wärmewirkung. Die Knieschiene läßt sich einfach anlegen. Der Heilungsprozeß ist gut kontrollierbar. Eine postoperative Wundversorgung ist möglich. Durch Verwendung geeigneter Materialien ist die Knieschiene schmutzunempfindlich.

Weiterentwicklungen der Erfindung sind Gegenstand der Unteransprüche.

Ausführungsbeispiele der Erfindung sind in den Zeichnungen dargestellt. Es zeigt
Fig. 1 eine Ansicht von oben auf die Innenseite der entfalteten Knieschiene,
Fig. 2 eine Ansicht von oben auf die Außenseite der entfalteten Knieschiene,
Fig. 3 einen senkrechten Längsschnitt gemäß Linie III-III in Fig. 1,
Fig. 4 in einer schaubildlichen Ansicht einen Abschnitt der Knieschiene mit dem Kniebeugehaltekissen und
Fig. 5 eine Seitenansicht des Kniebeugehaltekissens in natürlicher Größe.

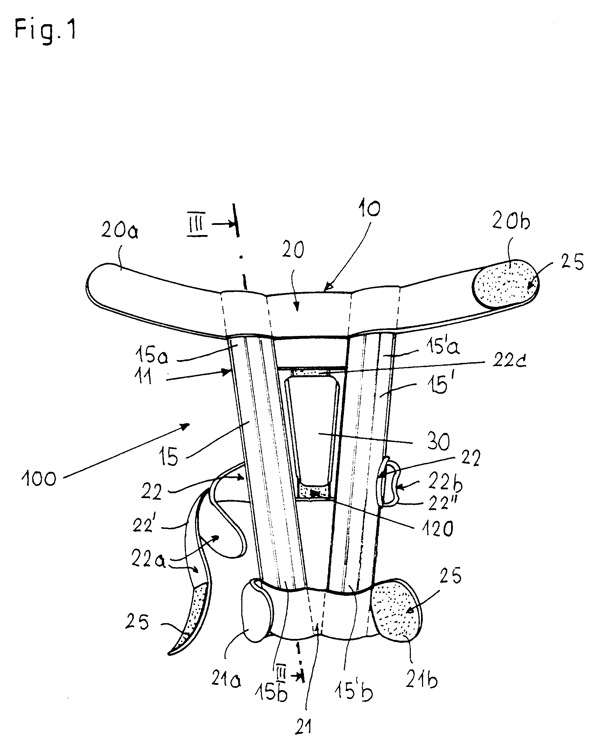

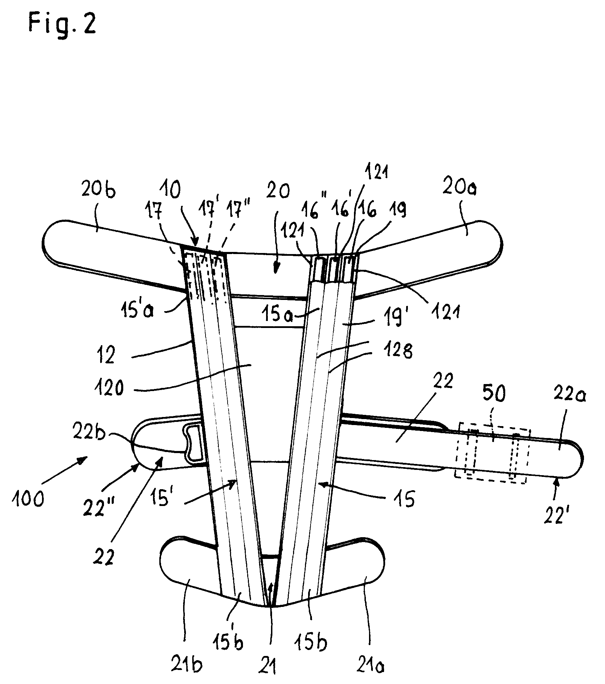

Gemäß Fig. 1 und 2 besteht die Knieschiene 100 aus einer am Bein eines Patienten anzulegenden und auf der Vorderseite des Beines zusammengehaltenen Hülse 10, die auf ihrer Innenseite 11 im Kniekehlenbereich des Beines ein bei angelegter Knieschiene 100 das Knie in einer vorgegebenen Beugehaltung von etwa 10° bis 20°, zweckmäßigerweise von 15° bis 20° oder von 15° oder 20°^{,} haltendes Kissen 30 aufweist. Dieses Kniebeugehalte- bzw. -polsterkissen 30 besteht aus einem formstabilen, kompressiblen und ein Rückstellvermögen aufweisenden Kunststoff, zweckmäßigerweise aus einem Schaumkunststoff. Als Schaumkunststoff werden offen- oder geschlossenzellige Schaumkunststoffe mit einem entsprechenden Verformungswiderstand eingesetzt. Bevorzugterweise werden Schaumkunststoffe aus PUR,PVC und PE eingesetzt. Auch andere geeignete,die gleichen Eigenschaften aufweisenden Materialien können eingesetzt werden. Die Hülse 10 zur Ausbildung einer flexiblen, jedoch stabilen Halbschale besteht aus zwei stabilen, eine hohe Eigensteifigkeit aufweisenden, den Beinkonturen anpaßbaren Seitenteilen 15,15', die an ihren beiden Enden 15a,15'a und 15b,15'b über je ein in der Länge verstellbares Verschlußband 20,21 miteinander und mittig über einen Verbindungssteg 120 verbunden sind. An bzw. auf dem mittigen Verbindungssteg 120, und zwar im Bereich zwischen den beiden Seitenteilen 15, 15' ist das Kissen 30 angeordnet, das in Knieschienenlängsrichtung verläuft und das zweckmäßigerweise etwa eine der Kniebreite des Beines des Patienten entsprechende Breite aufweist.Im Bereich des Verbindungssteges 120 sind zu dessen beiden Seiten,bevorzugterweise an den Außenrändern, die beiden Verschlußbandteile 22'22'' eines weiteren mittigen Verschlußbandes 22 angebracht, wobei sich die beiden Verschlußbandteile 22',22'' zu dem Verschlußband 22 ergänzen (Fig.1).

Nach einer Ausführungsform kann das Kissen 30 fest auf der Innenseite 22c des mittleren Verbindungssteges 120 der Knieschiene 100 angeordnet sein, so daß das Kissen 30 auf der Innenseite 11 der Knieschiene zu liegen kommt.

Zweckmäßigerweise ist jedoch nach einer weiteren Ausführungsform das Kissen 30 lösbar auf der Innenseite 22c des mittleren Verbindungssteges 120 der Knieschiene 100 angeordnet. Die Länge des mittleren Verbindungssteges 120 ist dabei gegenüber der Länge des Kissens 30 größer bemessen, so daß das Kissen 30 auf dem mittleren Verbindungssteg 120 lageveränderlich, d.h. in verschiedenen Positionen, gehalten werden kann (Fig.1,3 und 4). Durch die abnehmbare Anordnung des Kissens an dem mittleren Verbindungssteg besteht die Möglichkeit, die Knieschiene auch ohne Kissen einsetzen zu können, was immer dann der Fall sein wird, wenn das Bein in gestreckter Lage gehalten werden soll.

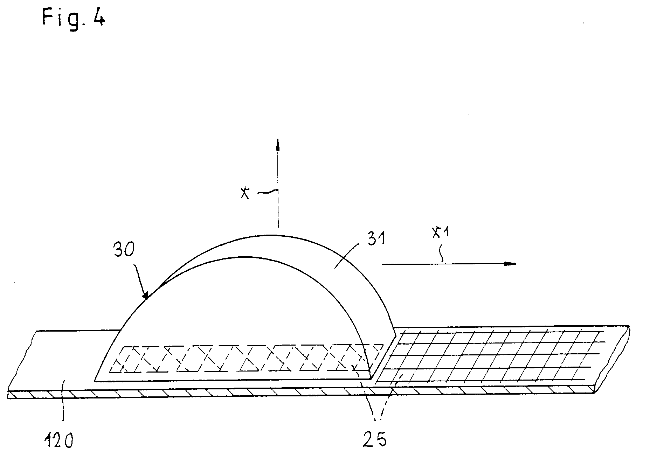

Um das Kissen 30 vom mittleren Verbindungssteg 120 in Pfeilrichtung X abnehmen zu können und wiederum auf dem mittleren Verbindungssteg 120 befestigen zu können, um das Kissen 30 auf diesem Verbindungssteg 120 in einer anderen Position benachbart zum oberen Ende oder zum unteren Ende der Knieschiene 100 befestigen zu können, ist zweckmäßigerweise eine klettverschlußartige Verbindung 25 vorgesehen (Fig.4). Diese klettverschlußartige Verbindung ist in an sich bekannter Weise ausgebildet. Anstelle einer klettverschlußartigen Verbindung 25 können auch anderweitig ausgebildete Verbindungsmittel eingesetzt werden, um das Kissen 30 von dem mittleren Verbindungssteg 120 abnehmen und in anderer Position wieder anordnen zu können, wobei die Lageveränderbarkeit durch den Pfeil X1 in Fig.4 angedeutet ist. Auf diese Weise ist eine Anpassung des Kissens 30 an den Beugebereich des Knies des Beines des Patienten möglich. Der Verbindungssteg 120 wird von einem entsprechend gestalteten Gewebezuschnitt gebildet.

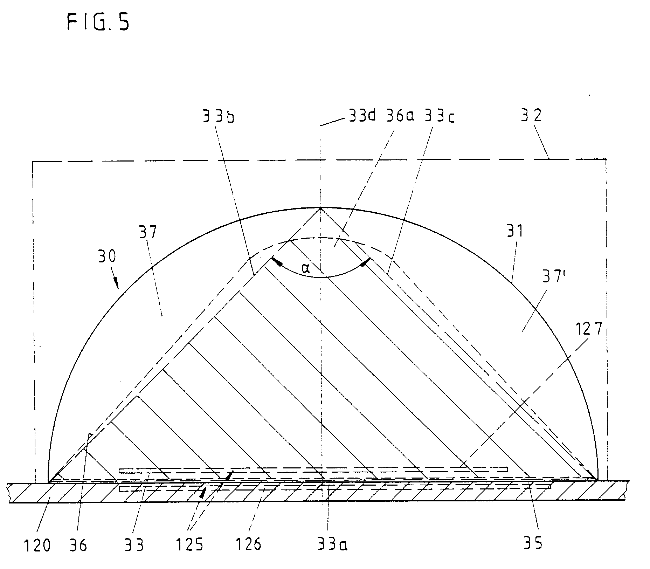

Nach einer weiteren Ausführungsform kann das Kissen 30 auch auf dem mittleren Verbindungssteg 120 mittels einer Magnetverbindung 125 gehalten sein, wobei dann der mittlere Verbindungssteg 120 mindestens einen Magneten 126 trägt, während die Auflagefläche 35 oder der der Auflagefläche 35 benachbarte Bereich des Kissens 30 mit einem plattenförmigen Zuschnitt 127 aus einem magnetisierbaren Material besteht. Auch mit einer derartigen Verbindung ist das Kissen 30 von dem mittleren Verbindungssteg 120 abnehmbar und in anderer Position auf diesem befestigbar. Um das Gewicht einer derartigen Magnetverbindung möglichst gering zu halten, ist es vorteilhaft, wenn an dem Verbindungssteg 120 in Abständen voneinander mehrere kleine Magneten 126 vorgesehen sind. Der Zuschnitt 127 aus magnetisierbarem Material kann dünnwandig und streifenförmig ausgebildet sein (Fig.5). Das dabei ausausgebildete Magnetfeld kann zur Unterstützung der Heilung nach operativen Eingriffen am Knie herangezogen werden.

Die Seitenteile 15,15' der Hülse 10 der Knieschiene 100 weisen als stabilisierende Elemente mindestens je einen in Knieschienenlängsrichtung verlaufenden rohrförmigen Stab 16,17 auf (Fig.2). Nach einer bevorzugten Ausführungsform nimmt jedes Seitenteil 15,15' der Hülse 10 drei rohrförmige Stäbe 16,16',16'' und 17,17',17'' auf, die parallel zueinander verlaufend sind und deren Enden löffelartig und abgeflacht ausgebildet sein können. Zur Aufnahme dieser rohrförmigen Stäbe 16,16',16'' und 17,17',17'' besteht jedes Seitenteil 15,15' der Hülse 10 aus einem doppelwandig ausgebildeten Gewebestreifen 19,19', dessen Innenraum durch Längsnähte 125 in eine der Anzahl der aufzunehmenden rohrförmigen Stäbe 16,16',16'' und 17,17',17'' entsprechende Anzahl von taschenförmigen Kammern 121 unterteilt ist.Zur Entnahme der Stäbe, z.B. zwecks Austausch gegen Stäbe mit anderen technischen und elastischen Eigenschaften, sind die Aufnahmetaschen für die Stäbe einseitig offen ausgebildet und mittels Verschlußlaschen verschließbar.

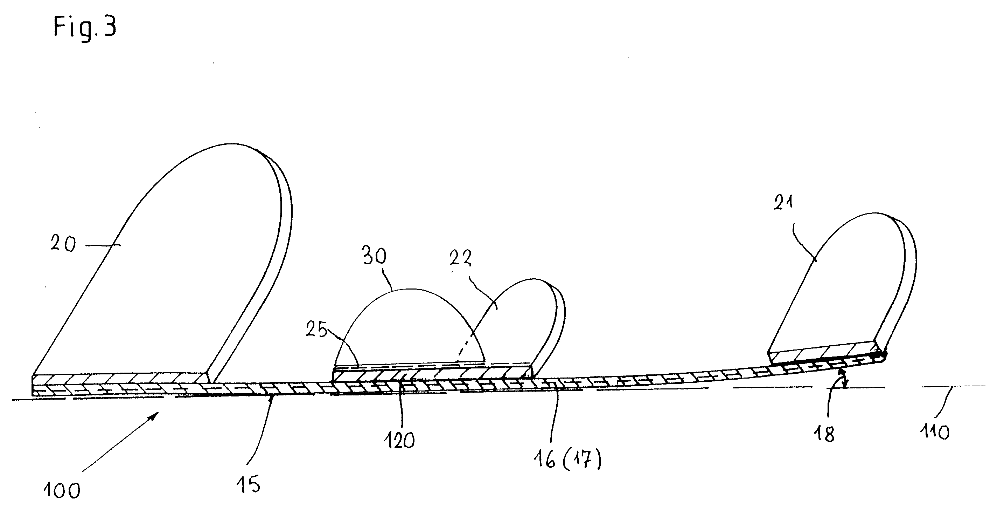

Diese rohrförmigen Stäbe 16,16',16'' und 17,17',17'' der Seitenteile 15,15' der Knieschiene 100 geben den Beugewinkel für das Knie nicht vor, sondern sind zweckmäßigerweise geradlinig mit einer Abwinkelung 18 im Bereich der unteren Enden 15b,15'b der Seitenteile 15,15' der Knieschiene 100, also im unteren Wadenbereich zur besseren Anpassung an die Anatomie ausgebildet, so daß die Seitenteile 15,15' in ihren unteren Bereichen gegenüber einer waagerechten Auflagefläche 110 leicht nach oben abgewinkelt sind (Fig.3). Des weiteren sind die rohrförmigen Stäbe 16, 16',16'' und 17,17',17'' mit kreisförmigen Querschnitten versehen, wobei jedoch auch andere geometrische Querschnittsformen vorgesehen sein können. Des weiteren bestehen die rohrförmigen Stäbe 16,16',16'' und 17,17',17'' aus einem Leichtmetall, zweckmäßigerweise aus Aluminium oder glasfaserverstärktem Kunststoff; auch eine Ausbildung der rohrförmigen Stäbe als Kohlefaserstäbe ist möglich. Wesentlich ist, daß die rohrförmigen Stäbe kein hohes Gewicht aufweisen. Auch vollwandig ausgebildete rohrförmige Stäbe können eingesetzt werden. Die bei der Knieschiene eingesetzten Stäbe sind trotz Beibehaltung einer hohen Eigensteifigkeit in einem gewissen Grade verformbar, um die Knieschiene den Konturen des Beines anpassen zu können.

Die beiden Seitenteile 15,15' der Hülse 10 sind V-förmig und konisch in Richtung zum Wadenbereich verlaufend ausgebildet (Fig.1 und 2). Der Verbindungssteg 120 zwischen den beiden Seitenteilen 15,15' weist ebenfalls eine V-Form auf. Das Kissen 30 ist dem Verlauf der Seitenteile 15,15' angepaßt und ist konisch sich nach unten in Richtung zu dem Verschlußband 21 verjüngend ausgebildet.

Die die beiden Seitenteile 15,15' der Hülse 10 miteinander verbindenden Verschlußbänder 20,21 und die beiden Verschlußbandteile 22',22'' des mittleren Verschlußbandes 22 sind an ihren Enden 20a,20b,21a,21b und 22a,22b mit Verschlüssen, zweckmäßigerweise mit Klettverschlüssen versehen, so daß eine Längenverstellbarkeit der Verschlußbänder 20,21,22 gegeben ist. Die die beiden Seitenteile 15,15' der Hülse 10 endseitig verbindenden Verschlußbänder 20,21 sind nach einer bevorzugten Ausführungsform innenwandseitig an den Seitenteilen 15, 15' befestigt, wobei das mittlere Verschlußband 22 mit seinen Verschlußbandteilen 22',22'' außenwandseitig an den Seitenteilen 15,15' gehalten ist (Fig.1 und 2). Um ein Einschnüren des angelegten mittleren Verschlußbandes 22 am Patientenbein zu verhindern, kann das Verschlußbandteil 22' zweiteilig ausgebildet sein. Das andere Verschlußbandteil 22'' ist bevorzugterweise als Schnalle , Gurtschnalle 22b ausgebildet. Ist das Verschlußbandteil 22' einteilig ausgebildet, dann ist auf das Verschlußbandteil 22' ein Polsterkissen 50 aufgeschoben (Fig.2).

Das Kissen 30 besteht aus einem etwa halbkreisförmigen Formkörper 31, der so bemessen ist, daß in dessen Längsquerschnittsfläche 32 die Fläche eines mit seiner Basis 33a auf der Kissenstandfläche 35 aufliegenden, gleichschenkligen und rechtwinkligen Dreiecks 33 mit einer dem Radius des halbkreisförmigen Formkörpers 31 entsprechenden Mittellinie 33d legbar ist. Die Katheten der Dreiecksfläche 33 sind mit 33b,33c bezeichnet. Die Basis 33a der Dreiecksfläche 33 bildet gleichzeitig die Auflagefläche 35 des Kissens 30. Der der Basis gegenüberliegende Winkel α beträgt in etwa 90° (Fig.5).

Nach einer weiteren Ausführungsform besteht der Formkörper 31 des Kissens 30 aus einem etwa dreieckförmigen Kern 36 und aus diesen im Seitenbereich abdeckenden polsterartigen und segmentartigen Abschnitten 37,37', wobei der Kern 36 eine gegenüber der Härte des Materials der polsterartigen Abschnitte 37,37' größere Härte aufweist. Diese polsterartigen Abschnitte 37,37' bestehen aus einem geeigneten Schaumkunststoff, wobei auch Silikonkautschuke eingesetzt werden können. Die Differenz zwischen der Härte der polsterartigen Abschnitte 37,37' und der Härte des Kerns 36 beträgt mindestens 10 Shore A, zweckmäßigerweise 20 Shore A. Das Material der polsterartigen Abschnitte 37,37' weist eine unterhalb 50 Shore A liegende Härte und das Material des Kerns 36 eine oberhalb 50 Shore A liegende Härte auf.

Der Formkörper 31 des Kissens 30 der Knieschiene 100 bzw. die polsterartigen Abschnitte 37,37', die abschnittsweise den Kern 36 des Formkörpers 31 umgeben, besteht aus einem weichen oder weichelastischen Material, wie Filz, Moosgummi, Neopren, Gummi oder aus viskoelastischem Silikonkautschuk oder aus einem elastisch, kompressiblen, durch Druck verformbaren Silikonkautschuk, z.B. mit einer Härte von 40 Shore A, einem Silikonschaum bzw. einem Kunststoffschaum mit einer Härte von 9 bis 13 Shore A oder einem kompressiblen, durch Druck verformbaren und ohne Sprungelastizität in seine Form sich rückbildenden Silikonkautschuk vom Typ eines nach dem Verfahren der Polyaddition vulkanisierenden Kaltkautschuks, der neben einer hohen Flexibilität eine unter 4 Shore A liegende Härte aufweist, wobei jedoch auch Silikonkautschuke eingesetzt werden können, deren Härte oberhalb von 4 Shore A liegt. Derartige viskoelastische Silikonkautschuke oder Materialien mit gleichen Elastizitätseigenschaften besitzen die Eigenschaft, bei angelegter Knieschiene 100 aufgrund der gleitenden Bewegung, ausgelöst durch Masseverschiebung,bei Druckanwendung oder bei Bewegungsabläufen, im Beugebereich des Knies massierend auf das Bein zu wirken. Besonders vorteilhaft ist es für die Herstellung des Formkörpers 31, ein Material zu wählen, welches viskoelastisch ist und aufgrund seiner elastischen Eigenschaften eine Massage bewirkt.

Besteht der Formkörper 31 aus einem Kern 36 mit einer Abpolsterung durch die Abschnitte 37,37', dann besteht dieser Kern 36, der auch eine andere geometrische Form aufweisen kann, aus einem harten oder inkompressiblen Kunststoff mit z.B. einer über 50 Shore A liegenden Härte und weist gegenüber den polsterartigen Abschnitten 37,37' eine weitaus größere Härte auf, um dem Kissen 30 eine gewisse Formstabilität zu geben. Als Material für den Kern 36 kommt natürlicher oder künstlicher Kautschuk oder Hartgummi infrage. So kann u.a. ein Chloropren-Polymerisat (Handelsname NEORPEN®) mit einer Härte von 50 Shore A, ein gummielastisches, vernetztes Polyurethan (Handelsname VULKOLAN®) mit einer Härte von 65 bis 90 Shore A, ein Silikonkautschuk mit einer Härte von 60 Shore A, ein Ethylen-Propylen-Dien-Kautschuk (EPDM) mit einer Härte von 80 Shore A oder ein Copolymerisat mit Acrylnitril (Handelsname PERBUNAN®) mit einer Härte von 70 Shore A, ferner ein Polyamid verwendet werden. Jedoch auch andere Kunststoffe oder Naturstoffabkömmlinge können zur Herstellung des Kerns 36 herangezogen werden. Wesentlich ist, daß der Kern 36 eine ausreichende Härte aufweist.

Um eine gute Abpolsterung eines mit einem Kern 36 versehenen Kissens 30 im Kniebeugebereich zu erhalten, ist der etwa dreieckförmige Kern 36 in seinem oberen Bereich 36a abgerundet, wobei die seitlichen polsterartigen Abschnitte 37,37' sich über diesen Bereich 36a erstrecken und einstückig ausgebildet sein können (Fig.5).

Der Formkörper 31 des Kissens 30 kann auch aus einer Hülle oder einem beutelartigen Körper mit einer dem Kissen 30 entsprechenden Form aus einer Kunststoffolie gebildet sein, deren Innenraum mit einem gelartigen Füllmittel aus einem Kunststoff, z.B. einem zähflüssigen Silikonöl, gefüllt ist, wobei das Füllmittel rückstellbare und viskoelastische Eigenschaften aufweist. Auch ein mit einem gasförmigen oder einem anderen flüssigen Medium gefülltes Kissen 30 kann zur Anwendung gelangen.

## Patentansprüche

1. Knieschiene mit einer am Bein anzulegenden, auf der Vorderseite des Beins zusammengehaltenen Hülse (10), dadurch gekennzeichnet,daß die Hülse (10) zur Ausbildung einer flexiblen, jedoch stabilen Halbschale aus zwei stabilen, eine hohe Eigensteifigkeit aufweisenden Seitenteilen (15, 15') besteht, die an ihren beiden Enden (15a, 15'a; 15b, 15'b) über je ein in der Länge verstellbares Verschlußband (20; 21) und mittig über einen Verbindungssteg (120) mit in dessen seitlichen Bereichen befestigten, sich zu einem mittleren Verschlußband (22) miteinander verbindbaren Verschlußbandteilen (22', 22'') miteinander verbunden sind.

2. Knieschiene nach Anspruch 1, dadurch gekennzeichnet, daß die Hülse (10) auf ihrer Innenseite (11) im Kniekehlenbereich des Patientenbeines ein bei angelegter Knieschiene (100) das Knie in einer vorgegebenen Beugehaltung von etwa 10° bis 20°, zweckmäßigerweise von 15° oder 20°, haltendes Kissen (30) aus einem formstabilen, kompressiblen und ein Rückstellvermögen aufweisenden Kunststoff, zweckmäßigerweise einem Schaumkunststoff, oder einem anderen geeigneten Material aufweist.

3. Knieschiene nach einem der Ansprüche 1 und 2, dadurch gekennzeichnet, daß an dem mittigen, bevorzugterweise aus einem Gewebezuschnitt bestehenenden Verbindungssteg (120) auf seiner Innenseite (11) im Kniekehlenbereich des Beines ein bei angelegter Knieschiene (100) das Knie in einer vorgegebenen Beugehaltung von etwa 10° bis 20° haltendes Kissen (30) aus einem formstabilen, kompressiblen und ein Rückstellvermögen aufweisenden Material angeordnet ist, wobei das Kissen (30) so ausgebildet ist, daß bei angelegter Knieschiene (100) das Knie in einer vorgegebenen Beugehaltung zweckmäßigerweise von 15^{o} bis 20° gehalten ist.

4. Knieschiene nach einem der Ansprüche 1 bis 3, dadurch gekennzeichnet, daß an den beiden Seitenteilen (15, 15') der Knieschiene (100) die sich zu einem mittleren Verschlußband (22) miteinander verbindbaren Verschlußbandteile (22',22'') befestigt sind.

5. Knieschiene nach einem der Ansprüche 1 bis 4, dadurch gekennzeichnet, daß das an dem mittleren Verbindungssteg (120) angeordnete Kissen (30) in Knieschienenlängsrichtung verläuft, in etwa eine der Kniebreite des Patientenbeines entsprechende Breite aufweist und aus einem etwa halbkreisförmigen Formkörper (31) besteht, der so bemessen ist, daß in dessen Längsquerschnittsfläche (32) die Fläche eines mit seiner Basis (33a) auf der Kissenstandfläche (35) aufliegenden, gleichschenkligen, rechtwinkligen Dreiecks (33) mit einer dem Radius des halbkreisförmigen Formkörpers (31) entsprechenden Mittellinie (33d) legbar ist.

6. Knieschiene nach einem der Ansprüche 1 bis 5, dadurch gekennzeichnet, daß der Formkörper (31) aus einem etwa dreieckförmigen Kern (36) und aus diesen in seinem Seitenbereich abdeckenden, polsterartigen und segmentartigen Abschnitten (37, 37') besteht, wobei der Kern (36) eine gegenüber der Härte des Materials der polsterartigen Abschnitte (37, 37') größere Härte aufweist, wobei die Differenz zwischen der Härte der polsterartigen Abschnitte (37, 37') und der Härte des Kerns (36) mindestens 10 Shore A, zweckmäßigerweise 20 Shore A, beträgt.

7. Knieschiene nach einem der Ansprüche 1 bis 6 , dadurch gekennzeichnet, daß das Kissen (30) fest auf der Innenseite (22c) des mittleren Verbindungssteges (120) angeordnet ist.

8. Knieschiene nach einem der Ansprüche 1 bis 6 , dadurch gekennzeichnet, daß das Kissen (30) lösbar auf der Innenseite (22c) des mittleren Verbindungssteges (120) angeordnet ist.

9. Knieschiene nach Anspruch 8 , dadurch gekennzeichnet, daß die Länge des mittleren Verbindungssteges (120) gegenüber der Länge des Kissens (30) größer bemessen ist, das auf dem mittleren Verbindungssteg (120) lageveränderbar gehalten ist.

10. Knieschiene nach einem der Ansprüche 1 bis 9, dadurch gekennzeichnet, daß das Kissen (30) auf dem mittleren Verbindungssteg (120) mittels einer mechanischen Halteeinrichtung, wie klettverschlußartige Verbinung (25) oder Magnetverbindung (125), wobei der mittlere Verbindungssteg (120) mindestens einen Magneten (126) trägt und die Auflagefläche (35) des Kissens (30) mit seinem plattenförmigen Zuschnitt (127) aus einem magnetisierbaren Material besteht, gehalten ist.

11. Knieschiene nach einem der Ansprüche 1 bis 10, dadurch gekennzeichnet, daß die Seitenteile (15, 15') der Hülse (10) als stabilisierende Elemente mindestens je einen in Knieschienenlängsrichtung verlaufenden, verformbaren, jedoch eine hohe Eigensteifigkeit aufweisenden Stab (16; 17) aufweisen.

12. Knieschiene nach einem der Ansprüche 1 bis 11, dadurch gekennzeichnet, daß jedes Seitenteil (15; 15') der Hülse (10) drei Stäbe (16,16';16''; 17, 17', 17'') aufweisen, die parallel zueinanderliegend sind.

13. Knieschiene nach einem der Ansprüche 1 bis 12, dadurch gekennzeichnet, daß jedes Seitenteil (15; 15') der Hülse (10) aus einem doppelwandig ausgebildeten Gewebestreifen (19, 19') besteht, dessen Innenraum durch Längsnähte (125) in eine der Anzahl der aufzunehmenden Stäbe (16, 16', 16''; 17, 17', 17'') entsprechende Anzahl von taschenförmigen Kammern (121) unterteilt ist.

14. Knieschiene nach einem der Ansprüche 1 bis 13, dadurch gekennzeichnet, daß die Stäbe (16, 16', 16''); 17, 17', 17'') eines jeden Seitenteils (15; 15') der Hülse (10) gerade verlaufend mit Abwinkelungen (18) im unteren Wadenbereich ausgebildet sind.

15. Knieschiene nach einem der Ansprüche 1 bis 14, dadurch gekennzeichnet, daß die Stäbe (16, 16' 16''; 17, 17', 17'') rohrförmig ausgebildet sind und einen kreisförmigen Querschnitt aufweisen.

16. Knieschiene nach einem der Ansprüche 1 bis 15, dadurch gekennzeichnet, daß die Stäbe (16, 16', 16''; 17, 17', 17'') aus einem Leichtmetall, zweckmäßigerweise aus Aluminium, glasfaserverstärktem Kunsttstoff bestehen oder als Kohlefaserstäbe ausgebildet sind.

17. Knieschiene nach einem der Ansprüche 1 bis 16, dadurch gekennzeichnet, daß die Stäbe (16, 16', 16''; 17, 17', 17'') vollwandig ausgebildet sind.

18. Knieschiene nach einem der Ansprüche 1 bis 17, dadurch gekennzeichnet, daß die beiden Seitenteile (15, 15') der Hülse (10) V-förmig und konisch in Richtung zum Wadenbereich verlaufend ausgebildet sind und daß der Verbindungssteg (120) und das Kissen (30) V-Form aufweisen.

19. Knieschiene nach einem der Ansprüche 1 bis 18, dadurch gekennzeichnet, daß die Verschlußbänder (20, 21, 22) an ihren Enden (20a, 20b; 21a, 21b; 22a, 22b) mit Verschlüssen, zweckmäßigerweise mit Klettverschlüssen, zur Längenveränderbarkeit versehen sind.

20. Knieschiene nach einem der Ansprüche 1 bis 19, dadurch gekennzeichnet, daß die beiden endseitig die beiden Seitenteile (15, 15') der Hülse (10) verbindenden Verschlußbänder (20, 21) innenwandseitig (11) an den Seitenteilen (15, 15') befestigt sind, wobei das mittlere Verschlußband (22) mit seinen beiden Verschlußbandteilen (22', 22'') außenwandseitig (12) an den Seitenteilen (15, 15') gehalten ist, an deren Außenrändern die beiden Verschlußbandteile (22', 22'') einendseitig befestigt sind.

## Claims

1. Knee brace with a sleeve (10) to be applied to the leg and held together on the front side of the leg,
characterized in that
the sleeve (10), in order to form a flexible but stable half shell, is comprised of two individual lateral portions (15,15') possessing a high inherent rigidity, which are interconnected at both their ends (15a, 15'a; 15b, 15'b) by means of one longitudinally adjustable closing strap each (20;21) and, centrally, by means of a connecting web (120) with closing strap portions (22', 22''), fastened within its lateral areas that are interconnectable so as to form a central closing strap (22).

2. Knee brace according to Claim 1,
characterized in that
the sleeve (10), on its inside (11), within the area of the hollow of the knee of the patient's leg, when the knee brace is applied, is provided with a pad (30) keeping the knee in a predetermined bent position of approximately 10° to 20°, but expediently of 15° to 20°, said pad being comprised of an inherently stable, compressible plastic possessing elastic recovery capacity, expediently a foamed plastic or some other suitable material.

3. Knee brace according to either Claim 1 or 2,
characterized in that,
on the center connecting web (120), preferably comprised of a fabric blank, on its inside (11), within the area of the hollow of the knee of the leg, when the knee brace (100) is applied,, a pad (30) is provided maintaining the knee in a predetermined position of approximately 10° to 20°, said pad being comprised of an inherently stable, compressible material possessing elastic recovery capacity, in which case the pad is constructed in such a way that, with the knee brace (100) applied, the knee is maintained in a predetermined bent position expediently ranging from 15° to 20°.

4. Knee brace according to any of Claims 1 to 3
characterized in that,
to the two lateral portions (15, 15') of the knee brace (100), the closing strap portions (22', 22'') which are interconnectable so as to form a center closing strap (22) are secured.

5. Knee brace according to any of Claims 1 to 4,
characterized in that
the pad (30) disposed on the center connecting web (120) proceeds in the longitudinal direction of the knee brace, possesses a width that corresponds approximately to the knee width of the patient's leg and is comprised of an approximately semi circular shaped member (31), which is dimensioned in such a way that, in the longitudinal cross-sectional area of the same (32), the surface of a right-angled isosceles triangle (33) resting with its bottom (33a) upon the pad floor space possessing a center line (33d) corresponding to the radius of the semicircular shaped member (31), can be laid.

6. Knee brace according to any of Claims 1 to 5,
characterized in that
the shaped member (31) is comprised of an approximately triangular core (36) and of pad-like and segment-like sections (37, 37') covering the same within its lateral area, in which case the core (36) possesses a hardness that is greater than the hardness of the of the material of the pad-like sections (37, 37'), in which case the difference between the hardness of the pad-like sections (37, 37') and the hardness of the core (36) is at least 10 Shore A, but expediently 20 Shore A.

7. Knee brace according to any of Claims 1 to 6,
characterized in that
the pad (30) is rigidly disposed in the inside (22c) of the center connecting web (120).

8. Knee brace according to any of Claims 1 to 6,
characterized in that
the pad (30) is detachably disposed on the inside (22c) of the center connecting web (120).

9. Knee brace according to Claim 8,
characterized in that,
the length of the center connecting web (120), in comparison with the length of the pad (30), is dimensioned so as to be greater, which is retained on the center connecting web so as to be positionally variable.

10. Knee brace according to any of Claims 1 to 9,
characterized in that
the pad (30) is retained on the center connecting web (120) with the aid of a mechanical retaining means, such as a Velcro fastener-like connection (25) or a magnetic connection (125), in which case the central connecting web (120) carries at least one magnet (126) and in that the contact area (35) of the pad (30) with its plate-shaped blank (127) is comprised of a magnetizable material.

11. Knee brace according to any of Claims 1 to 10,
characterized in that,
the lateral portions (15, 15') of the sleeve (10), as stabilizing elements, possess at least one rod (16; 17) each proceeding in the longitudinal direction of the knee brace, said rod being deformable but possessing a high inherent rigidity.

12. Knee brace according to any of Claims 1 to 11,
characterized in that
each lateral portion (15;15') of the sleeve (10) is provided with three rods (16, 16'; 16''; 17, 17', 17''), which are located parallelly to each other.

13. Knee brace according to any of Claims 1 to 12,
characterized in that
each lateral portion (15;15') of the sleeve (10) is comprised of a double-walled strip of fabric (19,19'), whose interior is subdivided by longitudinal seams (125) into a number of pocket-like chambers (121) corresponding to the number of rods to be accommodated (16, 16', 16''; 17, 17' 17'').

14. Knee brace according to any of Claims 1 to 13,
characterized in that
the rods (16, 16', 16''; 17, 17', 17'') of each lateral portion (15;15') of the sleeve (10) are constructed so ad to proceed in a straight line and possessing angled portions (18) within the lower calf region.

15. Knee brace according to any of Claims 1 to 14,
characterized in that
the rods (16,16',16''; 17,17',17'') are constructed in a tubular fashion and possess a circular cross section.

16. Knee brace according to any of Claims 1 to 15,
characterized in that
the rods (16,16',16'';17,17',17'') are comprised of a light metal, expediently of aluminum, glass fiber-reinforced plastic or are constructed in the form of carbon fiber rods.

17. Knee brace according to any of Claims 1 to 16,
characterized in that
the rods (16,16',16'';17,17',17'') are constructed so as to be solid.

18. Knee brace according to any of Claims 1 to 17,
characterized in that
the two lateral portions (15,15') of the sleeve (10) are constructed so as to be proceeding in a V-like and conical fashion in the direction toward the calf region and in that the connecting web (120) and the pad (30) possess a V-like configuration.

19. Knee brace according to any of Claims 1 to 18,
characterized in that
the closing straps (20,21,22), on their ends (20a, 20b; 21a,21b; 22a,22b), are provided with closing means, expediently with Velcro fastener tapes so as to ensure a longitudinal variability.

20. Knee brace according to any of Claims 1 to 19,
characterized in that
the two closing straps (20,21) which interconnect at their ends the two lateral portions (15,15') of the sleeve (10), are, on their internal wall (11), secured to the lateral portions (15,15'), while the central closing strfap (22), with its two closing strap portions (22',22''), is retained with the external wall (12) on the lateral portions (15,15'), on whose external edges the two closing strap portions (22',22'') are secured with one end.

## Revendications

1. Attelle de genou avec une gaine (10) à appliquer sur la jambe, maintenue sur la face antérieure de la jambe, caractérisée en ce que la gaine (10) est constituée, pour former une demi-coque flexible, tout en étant cependant stable, par deux parties latérales (15, 15') individuelles stables, qui présentent une grande rigidité propre, qui sont reliées l'une à l'autre à leurs deux extrémités (15a, 15'a ; 15b, 15'b) par respectivement une bande de fermeture (20 ; 21) de longueur réglable et au milieu par une entretroise de liaison (120) avec des parties de bande de fermeture (22', 22'') fixées dans les zones latérales de celle-ci, pouvant être reliées en une bande de fermeture du milieu (22).

2. Attelle de genou selon la revendication 1, caractérisée en ce que la gaine (10) présente, sur sa face intérieure (11) dans la zone du creux poplité de la jambe du patient, un coussin (30) qui, lorsque l'attelle de genou (100) est appliquée, maintient le genou dans une position de flexion prédéterminée d'environ 10° à 20°, de manière appropriée de 15° ou de 20°, constitué par une matière plastique de forme stable, compressible et présentant une reprise élastique, de manière appropriée en une matière plastique mousse ou une autre matière qui convient.

3. Attelle de genou selon l'une des revendications 1 et 2, caractérisée en ce qu'un coussin (30) en une matière de forme stable, compressible et présentant une reprise élastique, est placé sur l'entretoise de liaison (120) du milieu, qui est constituée de préférence par une pièce découpée en tissu, sur sa face intérieure (11) dans la zone du creux poplité de la jambe, coussin qui maintient le genou dans une position de flexion prédéterminée d'environ 10° à 20°, le coussin (30) étant configuré de telle manière que, lorsque l'attelle de genou (100) est appliquée, le genou est maintenu dans une position de flexion prédéterminée, de manière appropriée de 15° à 20°.

4. Attelle de genou selon l'une des revendications 1 à 3, caractérisée en ce que les parties de bande de fermeture (22', 22'') qui peuvent être reliées l'une à l'autre sont fixées sur les deux parties latérales (15, 15') de l'attelle de genou (100).

5. Attelle de genou selon l'une des revendications 1 à 4, caractérisée en ce que le coussin (30) placé sur l'entretoise de liaison du milieu (120) passe dans le sens longitudinal de l'attelle de genou, présente une largeur qui correspond à peu près à la largeur du genou de la jambe du patient et est constitué par un corps moulé (31) à peu près en forme de demi-cercle qui est dimensionné de telle manière que la surface d'un triangle (33) rectangle isocèle, prenant appui avec sa base (33a) sur la surface de base du coussin (35) peut être posé dans la surface de section longitudinale du coussin avec une ligne médiane (33d) qui correspond au rayon du corps moulé (31) en forme de demi-cercle.

6. Attelle de genou selon l'une des revendications 1 à 5, caractérisée en ce que le corps moulé (31) est constitué par un noyau (36) à peu près triangulaire et par des sections (37, 37') de type segment et de type bourrelet qui recouvrent celui-ci dans sa zone latérale, le noyau (36) présentant une dureté supérieure à la dureté de la matière des sections de type bourrelet (37, 37'), la différence entre la dureté des sections de type bourrelet (37, 37') et la dureté du noyau (36) étant d'au moins 10 Shore A, de manière appropriée de 20 Shore A.

7. Attelle de genou selon l'une des revendications 1 à 6, caractérisée en ce que le coussin (30) est placé de manière fixe sur la face intérieure (22c) de l'entretoise de liaison du milieu (120).

8. Attelle de genou selon l'une des revendications 1 à 6, caractérisée en ce que le coussin (30) est placé de manière amovible sur la face intérieure (22c) de l'entretoise de liaison du milieu (120).

9. Attelle de genou selon la revendication 8, caractérisée en ce que la longueur de l'entretoise de liaison du milieu (120) est dimensionnée plus grande que la longueur du coussin (30) qui est maintenu en position variable sur l'entretoise de liaison du milieu (120).

10. Attelle de genou selon l'une des revendications 1 à 9, caractérisée en ce que le coussin (30) est maintenu sur l'entretoise de liaison du milieu (120) au moyen d'un dispositif de maintien mécanique, comme une jonction de type fermeture autoagrippante (25) ou une jonction magnétique (125), l'entretoise de jonction du milieu (120) portant au moins un aimant (126) et la surface d'appui (35) du coussin (30) avec sa pièce découpée en forme de plaque (127) étant constituée par une matière magnétisable.

11. Attelle de genou selon l'une des revendications 1 à 10, caractérisée en ce que les parties latérales (15, 15') de la gaine (10) présentent, comme éléments stabilisants, au moins une baguette (16 ; 17) chacune qui passe dans le sens longitudinal de l'attelle de genou, qui est déformable et qui présente cependant une grande rigidité propre.

12. Attelle de genou selon l'une des revendications 1 à 11, caractérisée en ce que chaque partie latérale (15 ; 15') de la gaine (10) présente trois baguettes (16, 16' ; 16'' ; 17, 17', 17'') qui sont situées parallèles l'une à l'autre.

13. Attelle de genou selon l'une des revendications 1 à 12, caractérisée en ce que chaque partie latérale (15 ; 15') de la gaine (10) est constituée par une bande de tissu (19, 19') configurée à double paroi dont l'espace intérieur est divisé par des coutures longitudinales (125) en un nombre de chambres en forme de poches (121) qui correspond au nombre des baguettes à loger (16, 16', 16'' ; 17, 17', 17'').

14. Attelle de genou selon l'une des revendications 1 à 13, caractérisée en ce que les baguettes (16, 16', 16'' ; 17, 17', 17'') de chacune des parties latérales (15 ; 15') de la gaine (10) sont configurées en allant droit avec des coudures (18) dans la zone inférieure du mollet.

15. Attelle de genou selon l'une des revendications 1 à 14, caractérisée en ce que les baguettes (16, 16', 16'' ; 17, 17', 17'') sont configurées en forme de tuyau et présentent une section circulaire.

16. Attelle de genou selon l'une des revendications 1 à 15, caractérisée en ce que les baguettes (16, 16', 16'' ; 17, 17', 17'') sont configurées en un métal léger, de manière appropriée en aluminium, en matière plastique renforcée par des fibres de verre ou comme des baguettes de fibres de carbone.

17. Attelle de genou selon l'une des revendications 1 à 16, caractérisée en ce que les baguettes (16, 16', 16'' ; 17, 17', 17'') sont configurées en parois pleines.

18. Attelle de genou selon l'une des revendications 1 à 17, caractérisée en ce que les deux parties latérales (15, 16') de la gaine (10) sont configurées en forme de V et coniques dans le sens de la zone du mollet et que l'entretoise de liaison (120) et le coussin (30) présentent une forme en V.

19. Attelle de genou selon l'une des revendications 1 à 18, caractérisée en ce que les bandes de fermeture (20, 21, 22) sont pourvues, à leurs extrémités (20a, 20b ; 21a, 21b ; 22a, 22b) de fermetures, de manière appropriée de fermetures autoagrippantes pour pouvoir régler la longueur.

20. Attelle de genou selon l'une des revendications 1 à 19, caractérisée en ce que les deux bandes de fermeture (20, 21) qui relient du côté de l'extrémité les deux parties latérales (15, 15') de la gaine (10) sont fixées du côté de la paroi intérieure (11) sur les parties latérales (15, 15'), la bande de fermeture du milieu (22) étant maintenue avec ses deux parties de bande de fermeture (22', 22'') sur le côté de la paroi extérieure (12) sur les parties latérales (15, 15') sur les bords extérieurs desquelles les deux parties de bande de fermeture (22', 22'') sont fixées du côté de l'une des extrémités.
